# EUROPEAN PATENT APPLICATION

(11) **EP 4 822 701 A1**
(43) Date of publication of application: **14.10.2026**
(21) Application number: 25462005.7
(22) Date of filing: 08.04.2025
(51) Int. Cl.: G16B 30/00, G16B 45/00

(54) **COMPUTER-IMPLEMENTED METHOD, COMPUTER PROGRAM PRODUCT, DEVICE AND SYSTEM**

(71) Applicant: Omixon Biocomputing Kft., 1117 Budapest (HU)
(72) Inventor: Kliszki, Enikö, H-1117 Budapest (HU)
(74) Representative: Müller-Boré & Partner Patentanwälte PartG mbB

(57) **Abstract**

A computer-implemented method is provided. The method comprises: determining a first sequence based on a plurality of sequence reads; displaying, on a display device, a user interface, UI, including at least one second sequence comprising one or more first sequence reads of the plurality of sequence reads, wherein when the first and second sequences are aligned, at least one of the first sequence reads comprises at least one position that differs from a corresponding position of the first sequence, and wherein the at least one position are displayed such that such a position is identifiable from the other positions where the first and the second sequences are identical; and upon receiving an indication that one of the at least one second sequences is selected via the UI, displaying on the UI information about matched one or more reference sequences that match the selected second sequence.

## Description

The application relates to a computer-implemented method comprising: determining a first sequence based on a plurality of sequence reads; displaying, on a display device, a user interface, UI, including at least one second sequence comprising one or more first sequence reads of the plurality of sequence reads, wherein when the first and second sequences are aligned, at least one of the first sequence reads comprises at least one position that differs from a corresponding position of the first sequence, and wherein the at least one position are displayed such that such a position is identifiable from the other positions where the first and the second sequences are identical; and upon receiving an indication that one of the at least one second sequences is selected via the UI, displaying on the UI information about matched one or more reference sequences that match the selected second sequence. The application further relates to a computer program product comprising computer-readable instructions that, when loaded and run on a computer, cause the computer to perform the computer-implemented method. The application further relates to a device comprising one or more processors configured to perform the computer-implemented method. The computer-implemented method, the computer program product and/or the device may be used for genotyping for HLA (human leukocyte antigen), KIR (killer-cell immunoglobulin-like receptors), ABO blood group antigens, Rhesus, MICA/B (MHC Class I-related Chain A/B) or other immunoregulatory genes. For example, genotyping can be used in transplantation to determine donor-recipient compatibility by analyzing genes such as HLA genes, and other clinically relevant genes such as KIR for immunological compatibility, ABO blood group antigens, and MICA to minimize rejection risk and enhance long-term graft survival. As another example, genotyping can be used in transfusion to determine donor-recipient compatibility by analyzing, e.g., ABO blood group antigens, Rhesus, etc.

Existing systems for sequence analysis, in particular, genotyping, may display sequence reads obtained from a sequencing platform and/or sequences identified using the sequence reads. For example, a consensus sequence may be constructed from a plurality of sequence reads. The system may display the constructed consensus sequence and at least a part of the plurality of sequence reads used for constructing the consensus sequence. The part of the plurality of sequence reads may be mapped to the constructed consensus sequence. The consensus sequence may be used for querying a sequence database to identify one or more reference sequences that match, at least partially, the consensus sequence among the reference sequences comprised in the sequence database. In the present disclosure, "querying" may indicate accessing a database and retrieving desired information from the database. As an example, when querying a database comprising reference sequences using a sequence or a sequence read, for each position of the reference sequence the nucleotide base of the reference sequence at this position is compared with the nucleotide base of the sequence or the sequence read which is used for the query at the same position.

Further, a "reference sequence" may be understood as a DNA (deoxyribonucleic acid), RNA (ribonucleic acid) or protein sequence that serves as a standard for comparison. Reference sequences typically are known sequences, that are sequences of nucleotide bases in a certain order that are assigned a specific name.

Further, a sequence that "matches" another sequence may indicate that the sequence has identical nucleotide bases as the other sequence at corresponding positions. In an exact match or a perfect match, the nucleotide bases at all positions are identical. In a partial match at least some but not all nucleotide bases at all positions are identical, i.e. at some positions the nucleotide bases may be differing from each other. Further, in some cases, a sequence may have inserted and/or deleted positions with respect to another sequence, which are referred to as gaps or indels (insertions/deletions). In the present disclosure, the term, "match" or "matched", may be understood as relating to sequences being identical to a significant degree, for example, a proportion (e.g., percentage) of positions with identical nucleotide bases being higher than a specified threshold (e.g., 90%, 95%, etc.) among positions that are present in both sequences (e.g., if one of the two sequences being compared is shorter than the other, the positions included in the shorter sequence).

In some circumstances, it may be desired to identify one or more reference sequences that match one or more sequence reads of the plurality of sequence reads that are used for determining the first sequence. In an example, these can be sequence reads that are relied upon for constructing the consensus sequence. For example, some sequence reads that are related to the consensus sequence may have positions with nucleotide bases different from those at corresponding positions in the consensus sequence. In another example, when e.g. a reference sequence is obtained by querying a database using one or more sequence reads or the consensus sequence, differences between the remaining sequence reads and the reference sequence may occur.

Also, it is possible that inherent mismatch error rates occur during sequencing due to the limitations and technical variability of different sequencing platforms. These errors can introduce mismatches, insertions, or deletions when comparing a sequence read to a reference genome or sequence.

Examples of such errors may be base calling errors, PCR amplification errors, and homopolymer errors to name a few. Base calling errors, which may occur when the sequencer incorrectly identifies a nucleotide during read processing. PCR amplification errors, are errors during the PCR step, which can introduce mutations in amplified DNA, which then get sequenced. Adapter/Barcode errors, may include incorrect assignment of barcodes or adapters can lead to read misidentification or contamination. In homopolymer errors sequences with long stretches of the same nucleotide (e.g., AAAA or TTTTT) are prone to insertion/deletion errors.

Specifically the following errors may occur:
Mismatch is an error in which a nucleotide in the read differs from the reference. This error often is due to base-calling errors or sequence misinterpretation. In an example a reference is ATCG and the read is ATGG, thus there is a mismatch of C to G.

Insertion is an error, according to which one or more extra nucleotides are introduced during sequencing. As an example a reference is ATCG, where the read is ATCCG, i,e. an extra nucleotide base (C) is inserted.

Deletion is an error, where one or more nucleotides are lost in the read compared to the reference. As an example, a reference is ATCG and the sequence read is ATG, i,e, a nucleotide base is deleted, namely (C).

Sequencers may assign Phred scores to indicate confidence in base calls, wherein higher scores mean fewer errors. Also sequencers may apply error correction algorithms. Specifically when obtaining a consensus sequence from sequence reads, combining multiple reads from the same region can reduce errors by creating a consensus sequence.

Further analysis on such sequence reads may be desirable to identify the cause of the differences as compared to the consensus sequence or the reference sequence, respectively. This may be important since there may be a biological cause for these differences, or the differences may be a result of the analysis done. Specifically, such further analysis may indicate the reliability of the analysis results or imply whether or not the results are trustworthy or additional sequencing/assaying should be performed.

In such cases, a user may typically access a known website providing a sequence alignment tool, such as Nucleotide Basic Logical Alignment Search Tool (BLAST) of the National Center for Biotechnology Information, enter the one or more sequence reads of interest on the website and obtain information regarding the reference sequences provided by the sequence alignment tool for this sequence read.

Accordingly, there is a need for improving the performance of sequence read analysis, specifically of analysing at least part of the raw data obtained from a sequencing platform, and reducing the time and inconvenience associated with aspects of the sequence read analysis.

According to an aspect, the problem relates to improving the performance of analysis of sequences or sequence reads obtained by sequence platforms, in particular, facilitating genotyping.

The problem is solved by the features disclosed by the independent claims and/or one or more alternative embodiments described in this application. Further exemplary embodiments are defined by the dependent claims.

According to an aspect, a computer-implemented method is provided. The method comprises:
- determining a first sequence based on a plurality of sequence reads;
- displaying, on a display device, a user interface, UI, including at least one second sequence comprising one or more first sequence reads of the plurality of sequence reads, wherein when the first and second sequences are aligned, at least one of the first sequence reads comprises at least one position that differs from a corresponding position of the first sequence, and wherein the at least one position are displayed such that such a position is identifiable from the other positions where the first and the second sequences are identical; and
- upon receiving an indication that one of the at least one second sequences is selected via the UI, displaying on the UI information about matched one or more reference sequences that match the selected second sequence.

In the present disclosure, the term, "sequence reads", may indicate sequence reads of nucleotide sequences obtained from one or more samples, e.g., through high-throughput sequencing technologies using sequencing instruments or platforms. Examples of the sequencing instruments or platforms may include, but are not limited to, MiSeq i100 Series, NovaSeq X Series and NextSeq Series produced by Illumina as well as MinION, GridION, Promethlon 2 Solo and PromethlON produced by Oxford Nanopore Technologies. Thus, the sequence reads may be considered as "raw data" obtained as a result of sequencing the one or more samples. Each sequence read may comprise a fragment of DNA or RNA. In some exemplary embodiments, the sequence reads may be a portion of raw data from a sequencing platform. For example, the portion may be determined based on a filtering criteria relating to, e.g., noise percentage, signal quality and/or length or number of the sequence reads.

In the present disclosure, the term, "sequence", may indicate a full, assembled and/or validated DNA sequence, i.e. a subsequent, ordered arrangement of nucleotide bases. For example, a sequence may be obtained from a plurality of sequence reads (e.g., as the "first sequence" mentioned above) by, for example, assembling the plurality of sequence reads and/or querying a sequence database with the plurality of sequence reads. Further, for example, in some circumstances, a sequence may simply indicate a single sequence read or a group of sequence reads (e.g., as the "second sequence" mentioned above). For this disclosure, a sequence read is at least partially consisting of the raw data obtained from a sequencing platform. A sequence is the result of processing the sequence reads, wherein the processing may comprise different levels of complexity, e.g. merely grouping one or more sequence reads to one group or, querying a database with a sequence read or constructing a sequence from two or more sequence reads.

In the present disclosure, if not stated differently, the terms "first sequence read" and "sequence reads" may be used interchangeably. The terms "first" and "second" are merely for differentiating the different terminologies to avoid confusions and do not imply their traditional English meanings. That is "first" and "second" do not imply any order or ranking.

In the present disclosure position of a sequence read differs from a position of another sequence read or from the position of the first sequence, if the nucleotide bases at this position are different. One position of the second sequence differs from a position of the first sequence, if the nucleotide bases at this position are different.

In some exemplary embodiments, each of the first sequence reads may comprise at least one position that differs from the corresponding position of the first sequence.

In other exemplary embodiments, one or more of the at least one second sequence may have no differing position as compared to the first sequence, in other words, an exact match with the first sequence. A second sequence having an exact match with the first sequence comprises one or more sequence reads with all positions being identical to the corresponding positions of the first sequence when the first and second sequences are aligned. It is noted, however, that a second sequence may be shorter or longer than the first sequence. In case the second sequence is shorter or longer than the first sequence, an exact match may indicate that no differing position exists among the positions that are contained in both the first and second sequences. Thus, a position in the first or second sequence which has no corresponding position in the other sequence due to the difference in the lengths of the first and second sequences is not considered as a gap (i.e. insertion or deletion).

In some exemplary embodiments, the matched one or more reference sequences that match the selected second sequence may be obtained by querying a sequence database that comprises reference sequences. For example, the sequence database may be queried using the selected second sequence and the selected second sequence may be aligned against the reference sequences comprised in the sequence database to identify the matched one or more reference sequences within the reference sequences comprised in the sequence database. The alignment of the selected second sequence with the reference sequences comprised in the sequence database may be performed using an existing alignment algorithm or tool such as Minimap2, Basic Local Alignment Search Tool (BLAST), or the like.

In some exemplary embodiments, the UI may comprise a graphical UI (GUI). In other exemplary embodiments, the UI may comprise a command-line user interface.

In some examples the sequence reads and/or the second sequence(s) may be provided and displayed in the GUI in a predetermined order, i.e. the sequence reads and/or the second sequence(s) may be grouped. Various grouping schemes can be implemented for convenience or for visualization efficiency for the user. In some embodiments, the grouping is based on the order in which the sequence reads are received from the sequencing platform. As another example, sequence reads and/or the second sequence(s) may be ordered according to their nucleotide bases at a specific position. As an example the sequence reads may be ordered so that sequence reads having the same nucleotide bases at the given position are located subsequently, i.e. one below the other. The grouping may be automatic or triggered by a user action. In some embodiments, multiple grouping options are provided to a user, e.g., through a dropdown menu, GUI, command line, or other mechanisms, such that a user can organize the visualization according to their preference.

As an example, a user may select the position in the GUI, which is used for ordering, i.e. grouping, the sequence reads. The user specifically may select the position of the sequence read or the position of the first sequence or the position of the second sequence, which is used for ordering the second sequences or the sequence reads. For example, the sequences or sequence reads having the same value at the selected position are grouped next to each other for display. The user may also choose the order by selecting the nucleotide bases in an order desired by the user. As an alternative, the order of the nucleotide bases could be predefined starting with the nucleotide (or value) having the lowest number of occurrence at the given position, then the nucleotide having the next to the lowest number of occurrence at the given position and so forth until the nucleotide having the highest number of occurrence at the given position. As an alternative, the order could be the inverse to the above mentioned, starting with the nucleotide having the highest number of occurrence at the given position, then the nucleotide having the second highest number of occurrence at the given position and so forth until the nucleotide having the lowest number of occurrence at the given position.

In some exemplary embodiments, the indication that one of the at least one second sequences is selected may be included in a user input received via the UI.

The method according to the above-stated aspect can achieve efficient provision of information about one or more reference sequences that match a second sequence selected from sequences that comprise sequence reads with positions differing from corresponding positions of a first sequence that is determined based on a plurality of sequence reads. This information allows to quickly and efficiently identify the reason for the second sequence being different from the first sequence. By way of example, there could be a biological reason for second sequence at least partially not matching the first sequence, and a user might further investigate on this reason. Also, by way of another example, the reference sequence identified by the second sequence might be very similar to the first sequence, so that the user might identify that there is no problem with the analysis. As yet another example, in case the selected second sequence has an exact match with the first sequence but is shorter or longer than the first sequence, one or more reference sequences that match the selected second sequence may be different from the first sequence. Also in such a case, the information about the matched one or more reference sequences may provide one of the bases for the user to determine whether further analysis on the sequence reads should be carried out.

In some exemplary embodiments, the first sequence may be a consensus sequence constructed based on the plurality of sequence reads. The consensus sequence may represent a determination by a known algorithm of the most common and/or most likely identity for each position based on the plurality of sequence reads at that position. In some circumstances, two or more consensus sequences may be constructed based on the plurality of sequence reads. As a specific example, when the exemplary embodiments are used for HLA genotyping, two consensus sequences may be determined, each corresponding to an HLA allele in the sample(s). In case two or more consensus sequences are constructed, the at least one second sequence may be displayed for each of the two or more consensus sequences. In the exemplary embodiments where one or more consensus sequences are determined as the first sequence, for each consensus sequence, one reference sequence may be obtained that matches, at least partially, the consensus sequence by querying a sequence database and information regarding said one reference sequence may be provided. In the context of the specific example of HLA genotyping as stated above, said one reference sequence obtained for each consensus sequence may be an HLA allele comprised in the sequence database (e.g., IMGT/HLA database). The sequence database queried for said one reference sequence matching the consensus sequence may be the same sequence database that may be queried for determining the matched one or more reference sequences that match the selected second sequence. In other words, the same sequence database may be used for both matching of the consensus sequences and of the selected second sequence with the reference sequences. This can improve efficiency of providing information about the matched one or more reference sequences. Additionally or alternatively, the use of the same sequence database can ensure consistency of the results of both matching processes, since the results that are provided use the same terminology, which might not be the case, if a different sequence database is queried with the selected second sequence as compared to the sequence database that is queried with the consensus sequence. Further, the querying of the sequence database with each consensus sequence to determine said one reference sequence may be performed using the same alignment algorithm or tool (e.g., Minimap2, BLAST, or the like) that may be used for determining the matched one or more reference sequences that match the selected second sequence as described above. This can ensure that, when there are differences in the results (e.g., said one reference sequence matching the consensus sequence is different from the matched one or more reference sequences that match the selected second sequence), the differences arise from the sequences used for querying and are not due to different alignment algorithms (since the same alignment algorithm is used).

In some exemplary embodiments, two first sequences may be provided and each first sequence is a consensus sequence. The two first sequences may be different from each other.

In some exemplary embodiments, the first sequence may be a reference sequence identified based on querying a sequence database with each sequence read and selecting the most probable reference sequence within the sequence database based on the queries. Each sequence read may be each of a plurality of sequence reads received from a sequencing platform or instrument. The most probable reference sequence may be a reference sequence that is most likely to be present, or in other words, has the highest probability of being present, in the sample from which the plurality of sequence reads is obtained. In some circumstances, the most probable reference sequence may be determined by querying the sequence database with each sequence read and associating one reference sequence to each sequence read by matching each sequence read with the reference sequences and determining the most probable reference sequence from the associated reference sequences using a statistical method and/or a machine learning algorithm. By way of example, the most probable reference sequence may be the reference sequence of all the reference sequences identified that is matched most often with a sequence read of all the sequence reads. The querying using each sequence read and the matching of each sequence read with the reference sequences may be performed using the same alignment tool (e.g., Minimap2, BLAST, or the like) that may be used for determining the matched one or more reference sequences that match the selected second sequence as described above. Further, the sequence database queried for determining the most probable reference sequence may be the same sequence database that may be queried for determining the matched one or more reference sequences that match the selected second sequence. This can improve efficiency of providing information about the matched one or more reference sequences and/or can ensure consistency of the results of both matching processes.

In some exemplary embodiments, the first sequence may be a reference sequence identified based on querying a sequence database with each sequence read of a subset of all sequence reads, i.e. not all of the sequence reads and selecting the most probable reference sequence within the sequence database based on the queries. In other words, the first sequence may be a reference sequence identified based on querying a sequence database with a plurality of sequence reads, i.e. one by one of not all of the sequence reads and selecting the most probable reference sequence within the sequence database based on the queries. In some circumstances, the most probable reference sequence may be determined by querying the sequence database with each sequence read of the subset of all sequence reads and associating one reference sequence to each sequence read by matching each sequence read of the subset of all sequence reads with the reference sequences and determining the most probable reference sequence from the associated reference sequences using a statistical method and/or a machine learning algorithm. The querying using each sequence read of the subset of all sequence reads and the matching of each sequence read of the subset of all sequence reads with the reference sequences may be performed using the same alignment tool (e.g., Minimap2, BLAST, or the like) that may be used for determining the matched one or more reference sequences that match the selected second sequence as described above. Further, the sequence database queried for determining the most probable reference sequence may be the same sequence database that may be queried for determining the matched one or more reference sequences that match the selected second sequence. This can improve efficiency of providing information about the matched one or more reference sequences and/or can ensure consistency of the results of both matching processes.

In some exemplary embodiments two first sequences may be identified, i.e. two reference sequences may be identified and the two first sequences, that is the two reference sequences may be different from each other.

In some exemplary embodiments a second sequence may comprise or consist of one single sequence read. In some exemplary embodiments a second sequence may comprise or consist of a plurality of sequence reads or all sequence reads received from a sequencing platform that are associated with a corresponding first sequence.

In some exemplary embodiments, the information about the matched one or more reference sequences comprises one or more of: reference sequence gene names or identifiers, match accuracy rate, match error rate, or any information on unmatched positions.

In some exemplary embodiments, those second sequences that have a number of differing positions higher than a threshold number may be noise sequences of interest. The method according to the above stated aspect may further comprise automatically querying a sequence database with each of the noise sequences of interest to identify one or more reference sequences that match each noise sequence of interest and store information about the matched one or more reference sequences in association with the respective noise sequence of interest. As explained further below, a noise sequence of interest may exist as a result of measurement and/or analysis noise or error, e.g., from the sequencing platform or sequencing technologies, or exceptions with the sample, e.g., a sample from an individual having more than two alleles for each gene, such as an HLA loci, and/or contamination of one or more samples. Further, in such exemplary embodiments, displaying may comprise upon receiving indication that a noise sequence of interest is selected, identify and display the stored information associated with the selected noise sequence of interest. These exemplary embodiments can efficiently provide the information about the matched one or more reference sequences that match the selected noise sequence of interest since the information has already been stored.

As an alternative or in addition, a second sequence is a noise sequence of interest, if a position of the first sequence is identified as a noise position and the second sequence differs from the first sequence at the noise position. In such embodiments, a noise position is a position at which a noise level is above a predetermined noise threshold. Alternatively or additionally, a noise position is a position at which the distance of the noise level from a confidence level of the first sequence at this position is below a predetermined distance threshold. In such embodiments, the method may further comprise automatically calculating the noise level of each position of the first sequence and displaying at least a part of results of automatically calculating the noise level of each position of the first sequence such that one or more positions of the first sequence that are identified as noise positions or noise positions of interest are identifiable from other positions of the first sequence that are not identified as noise positions or noise positions of interest. For example, the noise positions may be displayed with a color different from that of other positions.

The noise threshold is a threshold that may be applied to determine, whether a position is considered to be relevant for having noise. For example, in some exemplary embodiments, as long as the noise level at a given position is below the noise threshold, the position is not considered relevant for assessing noise.

The distance threshold is a threshold that may be applied to determine, whether a position is considered to be relevant for having noise. For example, in some exemplary embodiments, as long as the difference of the noise level at a given position from the confidence level of the first sequence at this position, i.e. the distance of these two values, is larger than the distance threshold, the position is not considered relevant for assessing noise.

The noise level at a certain position is defined as the number of sequence reads having a nucleotide base at the given position different from the nucleotide base of any of the first sequences at this position, divided by the total number of sequence reads associated with the first sequence.

The noise threshold may be 15%, 20% or 25% or 30%.

The predetermined distance threshold may be 10%, 5% or 3%.

A confidence level of first sequence is a measure for the certainty that the nucleotide base of the first sequence, e.g. a consensus sequence or a determined reference sequence at this position is correct. In one example, the confidence level can be the number of sequence reads having the nucleotide base most common at this position among all sequence reads divided by the total number of sequence reads. In other words, the confidence level can be the number of sequence reads having the same nucleotide base as the first sequence at this position, e.g. the consensus sequence or the determined reference sequence at this position, divided by the total number of sequence reads associated with the first sequence.

In some exemplary embodiments, those second sequences that have a number of differing positions higher than a threshold number may be noise sequences of interest and/or those second sequences of interest, which may differ from the first sequence at the noise position may be noise sequences and the method may further comprise upon receiving the indication, automatically querying a sequence database with the selected noise sequence of interest to identify one or more reference sequences that match the noise sequence of interest.

In some exemplary embodiments, the at least one position that differs from the corresponding position of the first sequence may be a result of:
a measurement and/or analysis noise when creating the first sequence reads from one or more samples; and/or
the one or more samples.

As an example, the sample and/or the sequence reads may be such that there does not exist a reliable consensus sequence. If, e.g. the sample should result in exactly two consensus sequences, analysing the sequence reads could result in e.g. three equally good sequences for the two consensus sequences, or a third sequence that is less common, but is represented in sequence reads at or above a threshold frequency. One reason could be that there is a biological cause for this result, maybe a biological anomaly, resulting in three consensus sequences. In some cases, a third sequence from donor tissue may be represented, for an individual who previously received donor tissue or a donor organ. As an alternative, there could contamination of the sample. As a further reason, the measurement of the sequence reads could be faulty. Specifically, the measurement and/or analysis noise mentioned above may include sequencing errors.

In some exemplary embodiments, an instruction to display on the UI information about the matched one or more reference sequences that match the selected second sequence may be further received in connection with the indication that one of the at least one second sequence is selected via the UI. In some circumstances, the instruction may be received via a trigger element that is: a permanent element that is present within the UI during a whole time in which the UI is displayed on the display device, or a temporary element that appears within the UI in response to a particular user input with respect to the selected sequence read. In other circumstances, the instruction may be received in the form of a command line input to the UI.

In some exemplary embodiments, displaying on the UI the information about the matched one or more reference sequences that match the selected second sequence may comprise ranking the matched one or more reference sequences based on a percentage of a match of each one of the matched one or more reference sequences with respect to the selected second sequence. In such exemplary embodiments, identifiers for each of the matched one or more reference sequences may be displayed in an order according to the ranking. Further, the step of ranking the matched one or more reference sequences based on a percentage of a match of each one of the matched one or more reference sequences with respect to the selected second sequence may comprise: determining the percentage of the match of each one of the matched one or more reference sequences with respect to the selected second sequence and, optionally, determining an error rate for each one of the matched one or more reference sequences with respect to the match with the selected second sequence. The percentage of the match and/or the error rate for each one of the one or more reference sequences may be further provided.

In some exemplary embodiments, the reference sequences may be human leukocyte antigen (HLA) alleles. Further, in some exemplary embodiments, the sequence database may be an IPD-MHC database. The IPD-MHC database may comprise known HLA alleles and other sequences such as of killer-cell immunoglobulin-like receptors (KIRs). In other exemplary embodiments, the sequence database may be an IMGT/HLA database. Further examples of the sequence database may include, but are not limited to, GenBank of NCBI, EMBL-EBI, DDBJ (DNA Data Bank of Japan), RefSeq of NCBI, Ensembl, Gencode, MG-RAST, GOLD (Genomes OnLine Database), UniProt, PDB (Protein Data Bank), InterPro, Pfam, dbSNP of NCBI, ClinVar, OMIM (Online Mendelian Inheritance in Man), GEO (Gene Expression Omnibus), ArrayExpress, RNAcentral, TAIR (The Arabidopsis Information Resource), ViPR (Virus Pathogen Database), etc.

It is noted, however, that functions of any one of the various aspects and exemplary embodiments described herein do not depend on specific sequences.

In some exemplary embodiments, the plurality of sequence reads may be obtained from a single sample.

In some circumstances, any one of the method according to the above stated aspect and various exemplary embodiments may further comprise: grouping the at least one second sequence in a specified order for display in the UI, wherein the grouping is performed automatically or in response to a user input to trigger the grouping.

According to another aspect, a computer program product is provided. The computer program product comprises computer-readable instructions that, when loaded and run on a computer, cause the computer to perform the method according to any one of the above-stated aspect and various exemplary embodiments.

According to yet another aspect, a device is provided. The device comprises one or more processors configured to perform the method according to any one of the above-stated aspect and various exemplary embodiments.

In some exemplary embodiments, the device may further comprise one or more storage media connected to the one or more processors and configured to store a sequence database comprising reference sequences.

Specifically, the device may access a database. The database may be located physically on the device or it may be accessed via a network. The database may be a cloud-based database and/or a distributed database. The database may comprises the reference sequences. The database may be accessed automatically once the above mentioned process is initiated, specifically, once a second sequence is selected.

The device may carry out a query algorithm. In order to do so, the device may access or have a processor and necessary periphery. The processor may execute the above mentioned queries, specifically in the form of one or more computer-executable algorithms. The algorithm(s) may be executed on the device or in/by a remote processor controlled by the device. In other words, executing the algorithm(s) may be cloud-based. The algorithm may contain software instructions and may be stored on the device with the other software instructions associated with other steps or functionalities described in this disclosure, or may be stored separately.

The device may interact with user. For this the device may have a user interface, UI, specifically a graphical UI, GUI, such as a display, specifically a touch display.

In some exemplary embodiments, a system may comprise:
one or more processors configured to perform the method according to any one of the above-stated aspect and various exemplary embodiments;
one or more storage media connected to the one or more processors and configured to store a sequence database comprising reference sequences,
wherein the one or more processors may be further configured to receive the plurality of sequence reads from the sequencing machine.

The subject matter described in the present application can be implemented as a method or as a system, possibly in the form of one or more computer program products. The subject matter described in the present application can be implemented in a data signal or on a machine readable medium, where the medium is embodied in one or more information carriers, such as a CD-ROM, a DVD-ROM, a semiconductor memory, or a hard disk. Such computer program products may cause a data processing apparatus to perform one or more operations described in the present application.

In addition, subject matter described in the present application can also be implemented as a system including a processor, and a memory coupled to the processor. The memory may encode one or more programs to cause the processor to perform one or more of the methods described in the present application. In some examples, the system may be a general purpose computer system. In other examples, the system may be a special purpose computer system including an embedded system.

Details of one or more implementations are set forth in the exemplary drawings and description below. Other features will be apparent from the description, the drawings, and from the claims. It should be understood, however, that even though embodiments are separately described, single features of different embodiments may be combined to further embodiments.
Fig. 1 shows a functional block diagram of an exemplary system according to the present disclosure.
Fig. 2 shows a flowchart of an exemplary process performed by an exemplary genotyping system according to the present disclosure.
Fig. 3 shows an example of a GUI displayed by the exemplary genotyping system according to the present disclosure.
Fig. 4 shows another example of a GUI displayed by the exemplary genotyping system according to the present disclosure.
Fig. 5 shows an enlarged version of a portion of the GUI shown in Fig. 3.
Fig. 6 shows yet another example of a GUI displayed by the exemplary genotyping system according to the present disclosure.
Fig. 7 shows an enlarged version of a part of the GUI shown in Fig. 6.
Fig. 8 shows an exemplary hardware configuration of a computer that may be used to implement at least a part of a system according to the present disclosure.

In the following text, a detailed description of examples will be given with reference to the drawings. It should be understood that various modifications to the examples may be made. In particular, one or more elements of one example may be combined and used in other examples to form new examples.

In the following, exemplary embodiments are described in relation to genotyping of HLA genes. It should be noted, however, any one of various aspects and exemplary embodiments described herein may also be applied to genotyping of genes other than HLA genes such as KIR, ABO blood group antigens, MICA, etc.

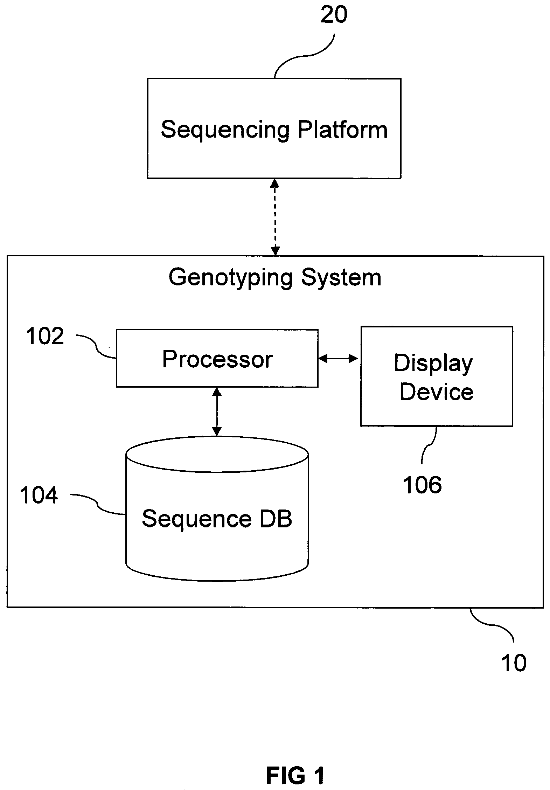

**Fig. 1** shows a functional block diagram of an exemplary system according to the present disclosure. The exemplary system shown in Fig. 1 comprises a genotyping system 10 and, optionally, a sequencing platform 20.

The genotyping system 10 may comprise a processor 102, a sequence database (DB) 104 and a display device 106. The processor 102 may be configured to perform one or more processes according to the present disclosure. The details of the processes that may be performed by the processor 102 will be described later below.

Further, the genotyping system 10 may comprise one or more storage media (not shown) connected to the processor 102 and configured to store any information that is necessary for the processor 102 to perform one or more processing. For example, the one or more storage media may store an alignment algorithm or tool used for querying the sequence DB 104 with a sequence and/or a sequence read.

The sequence DB 104 may comprise reference sequences. For example, the sequence DB 104 may comprise HLA alleles. In some exemplary embodiments, the sequence DB 104 may be an IMGT/HLA database, although the sequence DB 104 may also be other databases such as those described previously. For example, the sequence DB 104 may be implemented by storing the reference sequences in the one or more storage media of the genotyping system 10 stated above. In some exemplary embodiments, the sequence DB 104 may have the form of a file stored in the one or more storage media of the genotyping system 10.

The display device 106 may be configured to display information output by the processor 102.

In some exemplary embodiments, the genotyping system 10 may be implemented as a single device. In other exemplary embodiments, the genotyping system 10 may be implemented with more than one computing devices connected through a network such as the Internet. For instance, the genotyping system 10 may be implemented in a cloud computing environment, and use of the genotyping system 10 may be delivered to a user as a service.

The sequencing platform 20 may comprise a sequencing machine (not shown) configured to perform sequencing, where the nucleotide order of a given DNA or RNA fragment is determined. For example, the sequencing machine may be configured to perform measurements on one or more samples and obtain sequence reads of nucleotide sequences.

Further, the sequencing platform 20 may be configured to collect sequence reads obtained by the sequencing machine and store the collected sequence reads in a storage device and/or provide the collected sequence reads to another computing device. Each sequence read may be or contain in sequence, i.e. in a given subsequent order, nucleotides A, T, C, G. The data obtained from the sequencing machine may also include further information in addition to the order of nucleotides, such as metadata concerning the sequencing machine, the sample, the methods and processes used, etc. Examples of sequencing platform 20 may include, but are not limited to, sequencing platforms provided by Oxford Nanopore Technologies, Illumina, PacBio, etc. The sequencing platform 20 may be in communication with the genotyping system 10 via one or more wired and/or wireless networks. The processor 102 of the genotyping system 10 may receive, from the sequencing platform 20, sequence reads obtained from one or more samples by the sequencing platform 20. Alternatively, the sequence reads may be downloadable from the sequencing platform 20, and may be delivered to the genotyping system 10, e.g., through a computer readable medium.

It should be noted that the system shown in Fig. 1 is merely an example and a system according to the present disclosure may have configurations different from that of the exemplary system shown in Fig. 1. For example, the system according to the present disclosure does not need to comprise the sequencing platform 20, as long as the genotyping system 10 has access to the sequence reads obtained by the sequencing platform 20. For example, the display device 106 does not need to be comprised within the genotyping system 10 as shown in Fig. 1. The display device 106 may be, for example, in communication with the processor 102 of the genotyping system 10 but a separate device from the genotyping system 10. Such a form of implementation may be employed, for instance, in case the functions of the genotyping system 10 are provided within a web application that runs via a web browser. In such a case, the processor 102 and the database 104 of the genotyping system 10 may be implemented on one or more web servers and the display device 106 may be a display of a client device of a user, for example.

Further, although the genotyping system 10 shown in Fig. 1 comprises a single processor 102, the genotyping system 10 may comprise more than one processor 102 and said more than one processor 102 may be configured to perform one or more processes according to the present disclosure.

Further, for example, although the genotyping system 10 shown in Fig. 1 is in communication with the sequencing platform 20, in some circumstances, the genotyping system 10 does not necessarily be in communication with the sequencing platform 20. In such circumstances, the genotyping system 10 may access a storage device storing sequence reads that are obtained by the sequencing platform 20 to receive the sequence reads, wherein the storage device is not necessarily a part of the sequencing platform 20.

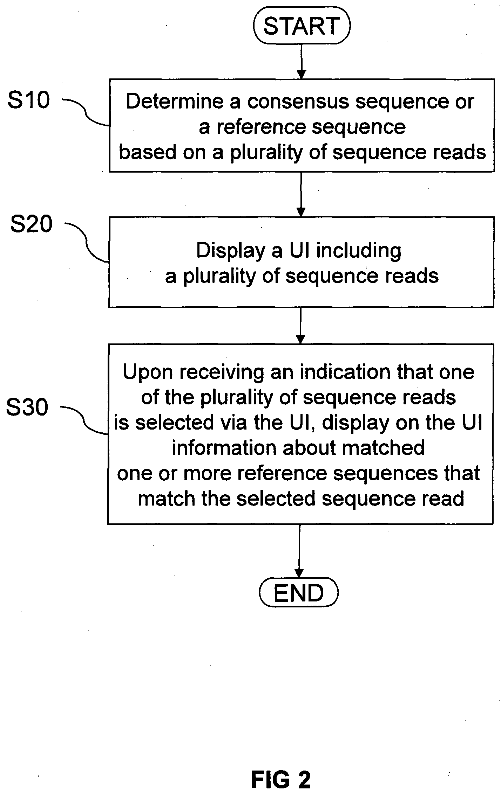

**Fig. 2** shows a flowchart of an exemplary process performed by the genotyping system 10. The processor 102 of the genotyping system 10 may start the exemplary process shown in Fig. 2 upon receipt of a user input instructing the genotyping system 10 to perform the exemplary process, for example.

In step S10, the processor 102 may determine a consensus sequence based on a plurality of sequence reads. The sequence reads may have been obtained from one or more samples and may be received from the sequencing platform 20. In a specific example where the exemplary process is used for HLA genotyping, two consensus sequences may be determined at step S10, each corresponding to an HLA allele in the sample(s). In this specific example, for each of the two consensus sequences, one reference HLA allele may be obtained that matches, at least partially, the consensus sequence by querying the sequence DB 104 comprising known HLA allele sequences. In this specific example, the sequence DB 104 may comprise the IMGT/HLA database. An alignment algorithm or tool such as Minimap2, BLAST, or the like may be used for mapping each consensus sequence to the HLA alleles comprised in the IMGT/HLA database. The alignment algorithm or tool may be stored in the one or more storage media of the genotyping system 10.

In alternative exemplary embodiments, a reference sequence is identified based on querying the sequence DB 104 with each sequence read of a subset of all sequence reads obtained or with each sequence read of all sequence reads obtained and selecting the most probable reference sequence within the sequence DB 104 based on the queries. In some circumstances, the most probable reference sequence may be determined by querying the sequence DB 104 with each sequence read and associating one reference sequence to each sequence read by matching each sequence read with the reference sequences and determining the most probable reference sequence from the associated reference sequences using a statistical method and/or a machine learning algorithm. In the matching of each sequence read with the reference sequences, an existing alignment tool such as Minimap2, BLAST, or the like may be used.

After step S10, the exemplary process may proceed to step S20.

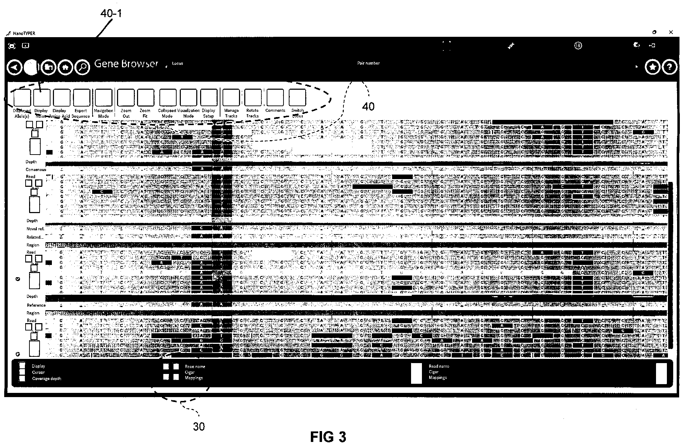

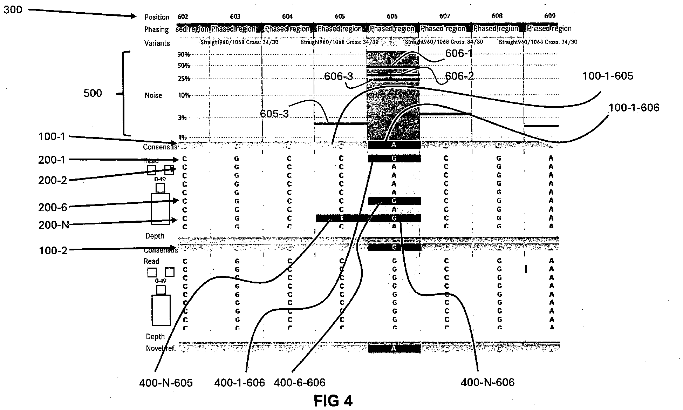

In step S20, the processor 102 may display, on the display device 106, a UI including a plurality of sequence reads. **Figs. 3** **and** **4** show examples of the UI displayed at step S20. These examples will be described more in detail later below. When the consensus sequences and the sequence reads are aligned, at least one of the sequence reads may comprise at least one position that differs from a corresponding position of the consensus sequence. In some circumstances, each of the sequence reads may comprise at least one position that differs from a corresponding position of the consensus sequence. The at least one position may be displayed in the UI such that such a position is identifiable from the other positions where the consensus sequence and the sequence reads are identical. In some exemplary embodiments, the UI displayed at step S20 may be a graphical UI (GUI). In other exemplary embodiments, the UI displayed at step S20 may be a non-graphical UI configured to receive command-line inputs from a user.

In some exemplary embodiments, those sequence reads that have a number, e.g., a percentage, of differing positions higher than a threshold number may be considered as noise sequence reads of interest, where the percentage may be calculated based on the ratio of the count of differing positions over the total count of sequence reads that are used for constructing the consensus sequence or for identifying the reference sequence. In some examples, the threshold is 5%, 10%, 15%, 20%, 25%, or higher. In some circumstances, the differing positions may be a result of a measurement and/or analysis noise when creating the sequence reads from one or more samples. Alternatively or additionally, the differing positions may be a result of the one or more samples. For instance, in the specific example involving the HLA genotyping, an individual may have, although rarely, more than two HLA alleles. In some cases, a sample may exhibit more than two alleles where an individual has previously received an organ or tissue donation. Constructing two HLA alleles based on sequence reads from a sample taken from such an individual may result in the noise sequences of interest.

**Figs. 3** **and** **4** show exemplary GUIs that may be displayed at step S20 of Fig. 2. In Fig. 3, the row indicated as "Consensus" shows at least a part of exemplary consensus sequences determined at step S10 of Fig. 2. Rows in the field labelled read shows exemplary sequence reads (not all displayed simultaneously but may be accessed/displayed when the UI is scrolled). Fig. 3 comprises various buttons. Specifically, buttons are shown in area 30 on the lower half of the GUI. Additional buttons are shown in area 40 on the upper half of the GUI. If the button 40-1 is selected, i.e. clicked by a mouse, the GUI changes to the view as shown in Fig. 4.

As an example, two consensus sequences 100-1 and 100-2 are shown in Fig. 4. In Figs. 3 and 4, rows immediately below each row indicated as the "Consensus" 100-1, 100-2 show examples of the sequence reads stated above with reference to step S20 of Fig. 2. In Fig 4, sequence reads 200-1, 200-2, 200-6, ..., 200-N are shown with respect to consensus sequence 100-1. Not the entire sequence reads are shown but merely the position 602, 603, ... 609 of the sequences are shown as can be seen from the top information line 300 in Fig. 4. A sequence read can have a large number of positions. By way of example, a sequence read may have 1000 or more positions. A sequence read may have up to 6000 or even 7000 positions. The sequence reads may have different lengths among themselves and also compared to the consensus sequence or the reference sequence associated with the sequence reads.

The exemplary sequence reads shown in the rows immediately below each row indicated as the "Consensus" comprise sequence reads belonging to a group of sequence reads from which the corresponding consensus sequence is constructed. As can be seen from Figs. 3 and 4, positions in the exemplary sequence reads differing from the corresponding positions of the corresponding consensus sequence are highlighted so that those differing positions are identifiable from the other positions that are identical to the corresponding positions of the corresponding consensus sequence.

Although highlighting is used, other mechanisms of making those differing positions identifiable can be implemented. For example, the characters (e.g., A, G, C, T) representing the nucleotide bases in those differing positions may be displayed with a color different from the characters in the corresponding positions of the corresponding consensus sequence. In the example shown in Fig. 4, two positions 100-1-605 and 100-1-606 are pointed out. The position 101-1-606 is identified by a different color background as compared to other positions. In addition, positions 400-1-606, 400-6-606, 400-N-606 and 400-N-605 are shown, which are part of the sequence reads 200-1, 200-6 and 200-N, respectively. At these positions the sequence reads 200-1, 200-6 and 200-N differ from the consensus sequence 100-1. The respective "values", i.e. the nucleotides of the sequence reads 200-1, 200-6 and 200-N differ from the "value", i.e. the nucleotide of the consensus sequence 100-1 at this position. Similarly at the position 310-1 the consensus sequence100-1 differs from the sequence read 200-N at position 410-N. Position 605 is identified, e.g. by applying a different color with respect to one or more other positions.

In Fig. 4 also the noise level with respect to the different positions are shown in area 500. At position 606 a high noise level, in the example of approximately 25% and above is shown. As a consequence, this position is highlighted, e.g. by a different of color with respect to a position having a lower noise level. At position 606 the consensus confidence level 606-1 of consensus sequence 1 is shown and the confidence level 606-2 of consensus sequence 2 is shown. Also shown is the noise level 606-3 of the sequence reads. The noise level is approximately 25%. Since the noise level 606-3 is above a predefined noise threshold or a commonly known platform introduced noise threshold, e.g. 20%, and/or since the noise level is close enough to the confidence level 606-1 of consensus sequence 1 and the confidence level 606-2 of consensus sequence 2, the position 606 is indicated as a position having noise of interest. As an alternative or in addition to the definition of a noise sequence of interest, any sequence read that has a nucleotide base at position 606 differing from the nucleotide base at position 606 of the consensus sequence 1 and differing from the nucleotide base at position 606 of the consensus sequence 2 may be considered a noise sequence of interest.

According to one example, the analysis of noise may be semi-automatic or automatic. Noise analysis may be carried out automatically by the system. A user may interact with the system by setting one or more thresholds, such as the noise threshold and/or distance thresholds. These thresholds maybe preset without the necessity of user-interaction. In case a position and/or a sequence read is identified, such a position and/or sequence read may be indicated. The indication may comprise that the position and/or the sequence read is displayed in a different color and/or a pop-up window appears and/or a message is provide, etc. displaying. It is also possible that a flag or other indications or alerts for the noise of interest on the screen is provided.

The above is an example only and not limited to the position 606 but the principle is applicable for every position of the consensus sequences or the determined reference sequences as well as for all sequence reads. Thus, this principle can be applied based on every position of the consensus sequences or the determined reference sequences for determining a noise sequence of interest.

At position 605 the noise level 605-3 is well below 3% and thus below the noise threshold mentioned above. As a consequence, i.e. since the noise level 605-3 is below the noise threshold, e.g. 3%, and/or since the noise level is spaced far enough from a confidence level (not shown) of consensus sequence 1 and the confidence level (not shown) of consensus sequence 2, the position 605 is not indicated as a position having noise of interest. That is, for position 605 not different display is provided while position 606 is displayed in a different color as compared to position 605.

The above is an example only and not limited to the position 605 but the principle is applicable for every position of the consensus sequences or the determined reference sequences as well as for all sequence reads. Thus, this principle can be applied based on every position of the consensus sequences or the determined reference sequences for determining a noise sequence of interest.

The noise threshold may be 20% or 25% or 30%. In some examples, if the noise level at a given position is above the noise threshold, then this position is highlighted so that specifically sequence reads that have a nucleotide base at this position that differs from the nucleotide base of the consensus sequence at this position can be selected and further analysed. If, in these examples, on the other hand the noise level at a given position is below the noise threshold, then this position is not highlighted and even if a sequence read has a nucleotide base at this position that differs from the nucleotide base of the consensus sequence at this position, nevertheless the sequence read might not be of interest for further analysis.

The noise level may be defined as the number of sequence reads having a nucleotide base at the given position different from the nucleotide base of any of the consensus sequences at this position, divided by the total number of sequence reads. For example, for position 606 of FIG. 4, the total unmatched sequence reads number with consensus sequence 100-1 is 500. The total number of sequence reads associated with the consensus sequence 100-1 is 2000. Accordingly, the noise level is 25%.

In another example, the noise level may be defined as the number of sequence reads having a nucleotide base at the given position different from the nucleotide base of the any of the determined preference sequences at this position, divided by the total number of sequence reads.

The sequence reads as shown are not provided in a specific order. However, the sequence reads may be provided in a predetermined order, i.e. they may be grouped. As an example, sequence reads may be ordered according to the nucleotide bases at a specific position. As an example the sequence reads may be ordered so that sequence reads having the same nucleotide bases at the given position are subsequently, one below the other. In the example shown, if the ordering is selected based on position 606, e.g. by a mouse-click, specifically a right mouse click, then from the first line downwards, all the sequence reads having a nucleotide base "G" at position 606 are displayed subsequently. Next will be all sequence reads having the nucleotide base "A", and so forth. This way of displaying is not limited to position 606 and nucleotide bases "G" and "A". Rather, this principle is generally applicable to every position and every nucleotide base. Specifically, a user may select the position, which is used for ordering, i.e. grouping, the sequence reads. The user may also choose the order by selecting the nucleotide bases in an order desired by the user. As an alternative, the order of the nucleotide bases could be predefined starting with the nucleotide having the lowest number of occurrence at the given position, then the nucleotide having the next to the lowest number of occurrence at the given position and so forth until the nucleotide having the highest number of occurrence at the given position. As an alternative, the order could be the inverse to the above mentioned, starting with the nucleotide having the highest number of occurrence at the given position, then the nucleotide having the second highest number of occurrence at the given position and so forth until the nucleotide having the lowest number of occurrence at the given position. The grouping criteria as described above are merely examples of how the grouping of the sequence reads can be performed and other grouping criteria may be employed. Further, in some exemplary embodiments, a user may select which grouping criteria to employ for grouping the sequence reads. In such exemplary embodiments, multiple options of grouping criteria such as the ones described above may be provided to the user (e.g., in the UI as buttons, a drop-down menu, command line, etc.) so that the user can select the desired grouping criteria according to which the grouping of the sequence reads is performed.

The nucleotide base shown at position 400-N-605 is statistically insignificant, since it is located at a position with very low noise level 605-3.

Referring again to Fig. 2, after step S20, the exemplary process may proceed to step S30.

At step S30, the processor 102 may, upon receiving an indication that one of the plurality of sequence reads is selected via the UI, display on the UI information about matched one or more reference sequences that match the selected sequence read. The information about the matched one or more reference sequences displayed on the UI at step S30 may comprise one or more of: reference sequence gene names or identifiers, match accuracy rate, or match error rate.

The indication received at step S30 may be included in a user input received via the UI. For example, a user may select one of the exemplary sequence reads via the exemplary GUI shown in Fig. 3 or 4. Further, for example, in case the UI comprises a non-graphical UI, the user may input a command-line selecting one of the sequence reads.

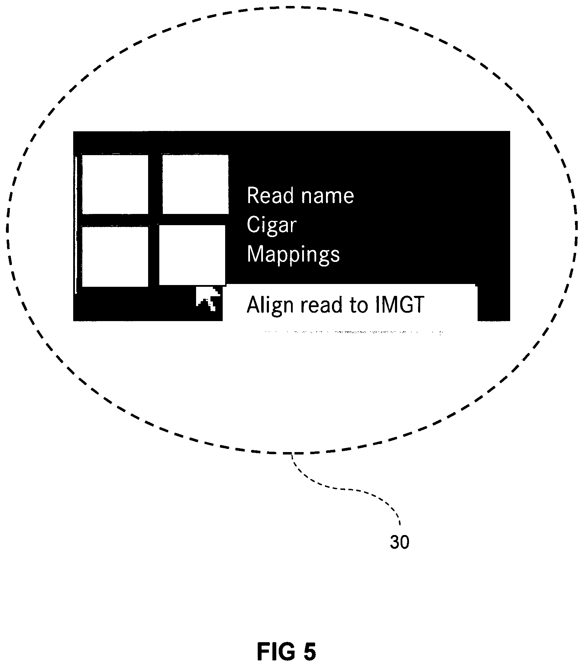

In some exemplary embodiments, an instruction to display, on the UI, information about the matched one or more reference sequences that match the selected sequence read may be further received in connection with the indication received at step S30 of Fig. 2. In some circumstances, the instruction may be received via a trigger element that is a permanent element that is present within the UI during a whole time in which the UI in response to a particular user input with respect to the selected sequence read. An area 30 indicated with a dashed line in the exemplary GUI shown in Fig. 3 comprises an example of a permanent trigger element, being a button that can be activated e.g. with a mouse click. **Fig. 5** shows an enlarged version of the area 30 shown in Fig. 3. A bottom right square element is the exemplary permanent trigger element, which, as mentioned above, can be a button. The button may comprise a name and/or written description. Metadata concerning the button, e.g. relating to its function, my be provided when hovering over the button or by a right-mouse-click on the button. For example, when a user clicks on the exemplary GUI of Figs. 3 and 5 after selecting a sequence read within the GUI, the selection of the sequence read and the instruction to display on the UI information about the matched one or more reference sequences that match the selected sequence read may be received by the processor 102. In other exemplary embodiments, the trigger element may be a temporary element that appears within the UI in response to a particular user input with respect to the selected sequence read. An example of such a temporary trigger element may be an option shown in a drop-down menu when a user "right-clicks" the selected sequence read on the GUI. In yet other exemplary embodiments where the UI comprises a non-graphical UI, the instruction may be received in the form of a command-line input to the UI.

Referring again to Fig. 2, the matched one or more reference sequences that match the selected sequence read displayed at step S30 may be obtained by querying the sequence DB 104. In some exemplary embodiments, the processor 102 may query the sequence DB 104 with the selected sequence read in response to receiving the indication of the selected sequence read. In other exemplary embodiments, the processor 102 may automatically query the sequence DB 104 with each of the noise sequences of interest prior to receiving the indication of the selected sequence read. In such exemplary embodiments, for example, when a noise sequence of interest is identified, which may be a single sequence read, i.e. a line in Fig. 3 and Fig. 4 due to having a number of the differing positions higher than the threshold number, the processor 102 may, for example, query the sequence DB 104 with the identified noise sequence of interest to identify one or more reference sequences that match the identified noise sequence of interest and store information about the matched one or more reference sequences. In such exemplary embodiments, upon receipt of the indication of the selected sequence read which is one of the noise sequences of interest, the processor 102 may identify and display the stored information associated with the selected one of the noise sequences of interest.

In either case of querying the sequence DB 104 in response to receiving the indication of the selected sequence read or of querying the sequence DB 104 with each noise sequence of interest prior to receiving the indication of the selected sequence read as described above, the sequence DB 104 may be used not only for identifying the matched one or more reference sequences that match the selected sequence read but also for determining the reference sequences that match each consensus sequence or for determining the reference sequence at step S10. In other words, for determining the matched one or more reference sequences that match the selected sequence read, the sequence DB 104 that is already available for the genotyping system 10 to determine the reference sequence at step S10 (or the reference sequence corresponding to each consensus sequence) may be queried. Thus, for determining the matched one or more reference sequences that match the selected sequence read, it may not be necessary to access a third-party sequence database that is separate from the genotyping system 10. This can enable significantly faster provision of the information about the matched one or more reference sequences that match the selected sequence read. Moreover, since the same sequence DB 104 is used for both of the matching processes, the results of both of the matching processes may be consistent. Further, the same alignment tool (e.g., Minimap2, BLAST, or the like) may be used for matching the selected sequence read with the reference sequences in the sequence DB 104 and for determining the reference sequence at step S10 (or the

In some exemplary embodiments, step S30 may further comprise ranking the matched one or more reference sequences based on a percentage of a match of each one of the matched one or more reference sequences with respect to the selected sequence read. In such exemplary embodiments, identifiers for each of the matched one or more reference sequences may be displayed in the UI in an order according to the ranking. Further, the step of ranking the matched one or more reference sequences based on a percentage of a match of each one of the matched one or more reference sequences with respect to the selected sequence read may comprise: determining the percentage of the match of each one of the matched one or more reference sequences with respect to the selected sequence read and, optionally, determining an error rate for each one of the matched one or more reference sequences with respect to the match with the selected sequence read. The percentage of the match and/or the error rate for each one of the one or more reference sequences may be provided in the UI at step S30. The ranking and displaying according to the ranking may present the information about the matched one or more reference sequences in a more relevant and streamlined way.

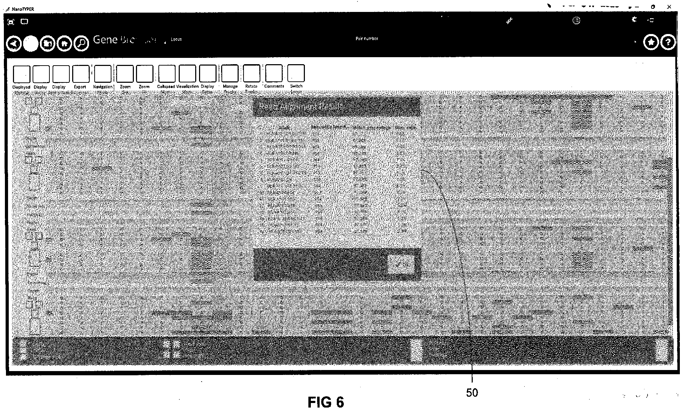

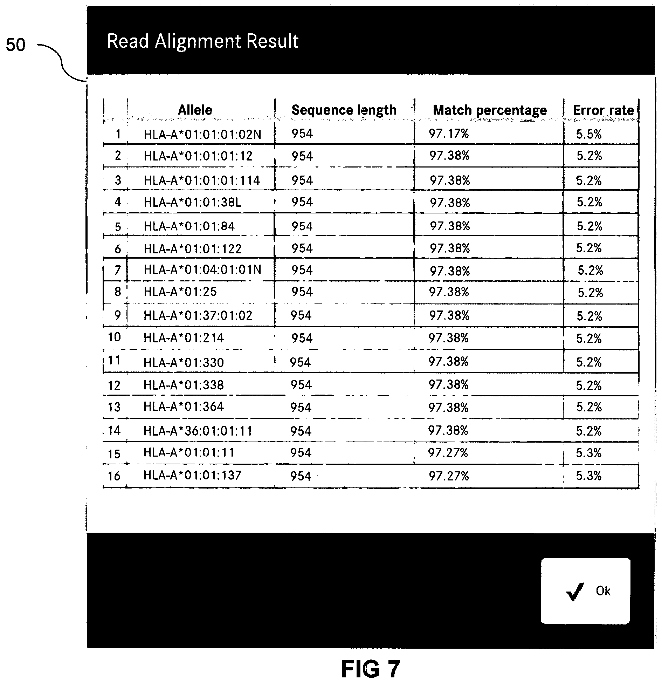

**Fig. 6** shows an exemplary GUI displayed at step S30 of Fig. 2. The exemplary GUI includes an exemplary window 50 including the information about the matched one or more reference sequences that match the selected sequence read, ranked according to the ranking step as described above. **Fig. 7** shows an enlarged version of the exemplary window 50 shown in Fig. 6.

Referring again to Fig. 2, the display of the information about the matched one or more reference sequences that match the selected sequence read may provide further information useful for genotyping. For instance, in the specific example of HLA genotyping, the matched one or more reference sequences that match the selected sequence read which is one of the noise sequences of interest can facilitate more accurate and/or fast identification of the HLA alleles of the sample from which the sequence reads are obtained as compared to prior art systems and methods, according to which the sequence read of interest is used for querying an external, different database that may not be accessible by the same alignment algorithm or tool

In some exemplary embodiments, step S30 may further comprise ranking the matched one or more reference sequences based on a percentage of a match of each one of the matched one or more reference sequences with respect to the selected sequence read. In such exemplary embodiments, identifiers for each of the matched one or more reference sequences may be displayed in the UI in an order according to the ranking. Further, the step of ranking the matched one or more reference sequences based on a percentage of a match of each one of the matched one or more reference sequences with respect to the selected sequence read may comprise: determining the percentage of the match of each one of the matched one or more reference sequences with respect to the selected sequence read and, optionally, determining an error rate for each one of the matched one or more reference sequences with respect to the match with the selected sequence read. The percentage of the match and/or the error rate for each one of the one or more reference sequences may be provided in the UI at step S30. The ranking and displaying according to the ranking may present the information about the matched one or more reference sequences in a more relevant and streamlined way.

**Fig. 6** shows an exemplary GUI displayed at step S30 of Fig. 2. The exemplary GUI includes an exemplary window 50 including the information about the matched one or more reference sequences that match the selected sequence read, ranked according to the ranking step as described above. **Fig. 7** shows an enlarged version of the exemplary window 50 shown in Fig. 6.

Referring again to Fig. 2, the display of the information about the matched one or more reference sequences that match the selected sequence read may provide further information useful for genotyping. For instance, in the specific example of HLA genotyping, the matched one or more reference sequences that match the selected sequence read which is one of the noise sequences of interest can facilitate more accurate and/or fast identification of the HLA alleles of the sample from which the sequence reads are obtained as compared to prior art systems and methods, according to which the sequence read of interest is used for querying an external, different database that may not be accessible by the same alignment algorithm or tool that is used for determining the reference sequence from the plurality of sequence reads (e.g., at step S10) or for determining a reference sequence that matches the consensus sequence. Particularly in case the sample contains more than two HLA alleles, identifying and displaying the matched one or more reference sequences that match the selected one of the noise sequences of interest can lead to faster identification of the third and further HLA alleles contained in the sample in addition to the two HLA alleles that may be identified using the two consensus sequences. Moreover, since the same sequence DB 104 is used for all of the matching processes, the results of all of the matching processes may be consistent.

After step S30, the exemplary process may end.

In exemplary embodiments, if the result of the matching of the selected sequence read provides a result that deviates from the consensus sequence or the determined reference sequence, an alert maybe provided. This may be the case, if e.g. the allele identified in the list e.g. shown in Fig. 7 is not of a group of predefined alleles. This group of predefined alleles may be alleles that may be expected with a certain degree of certainty based on the consensus sequence or the determined reference sequence. In addition or as an alternative, an alert may be provided, if e.g. the allele identified in the list e.g. shown in Figure 7 is of a group of predefined alleles. This group of predefined alleles may be alleles that may be excluded with a certain degree of certainty based on the consensus sequence or the determined reference sequence. In addition or as an alternative, if the result of the matching of the selected sequence read provides a result that deviates from the consensus sequence or the determined reference sequence, an alert may be provided.

The alert may be of any kind conventionally know, such as a pop-up window, a change in color in given window, display of a text message, sending an email message, etc.

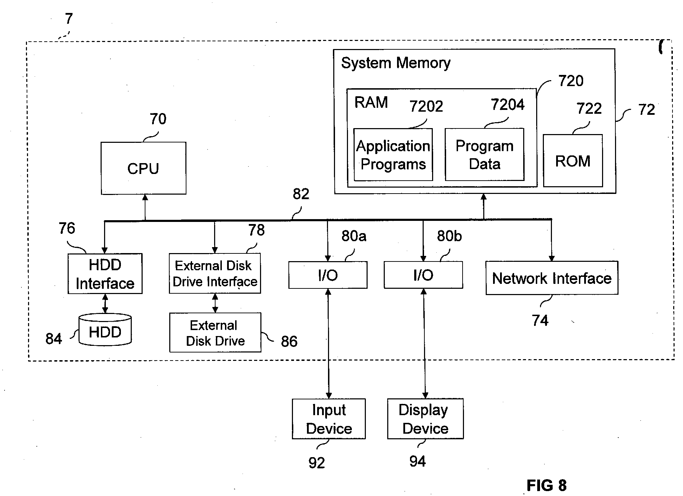

**Fig. 8** shows an exemplary hardware configuration of a computer that may be used to implement at least a part of the system as described above. For example, at least a part of the genotyping system 10 shown in Fig. 1 may be implemented with the computer 7 shown in Fig. 8. The computer 7 shown in Fig. 8 includes a central processing unit (CPU) 70, a system memory 72, a network interface 74, a hard disk drive (HDD) interface 76, an external disk drive interface 78 and input/output (I/O) interfaces 80. These components of the computer are coupled to each other via a system bus 82. The CPU 70 may perform arithmetic, logic and/or control operations by accessing the system memory 72. The system memory 72 may store information and/or instructions for use in combination with the CPU 70. The system memory 72 may include volatile and non-volatile memory, such as a random access memory (RAM) 720 and a read only memory (ROM) 722. A basic input/output system (BIOS) containing the routines that helps to transfer information between elements within the computer 7, such as during start-up, may be stored in the ROM 722. The system bus 82 may be any of several types of bus structures including a memory bus or memory controller, a peripheral bus, and a local bus using any of a variety of bus architectures.

The computer may include a network interface 74 for communicating with other computers and/or devices via a network.

Further, the computer may include a hard disk drive (HDD) 84 for reading from and writing to a hard disk (not shown), and an external disk drive 86 for reading from or writing to a removable disk (not shown). The removable disk may be a magnetic disk for a magnetic disk drive or an optical disk such as a CD ROM for an optical disk drive. The HDD 84 and the external disk drive 86 are connected to the system bus 82 by a HDD interface 76 and an external disk drive interface 78, respectively. The drives and their associated computer-readable media provide non-volatile storage of computer-readable instructions, data structures, program modules and other data for the general purpose computer. The data structures may include relevant data for the implementation of the exemplary method and its variations as described herein. The relevant data may be organized in a database, for example a relational or object database.

Although the exemplary environment described herein employs a hard disk (not shown) and an external disk (not shown), it should be appreciated by those skilled in the art that other types of computer readable media which can store data that is accessible by a computer, such as magnetic cassettes, flash memory cards, digital video disks, random access memories, read only memories, and the like, may also be used in the exemplary operating environment.

A number of program modules may be stored on the hard disk, external disk, ROM 722 or RAM 720, including an operating system (not shown), one or more application programs 7202, other program modules (not shown), and program data 7204. The application programs may include at least a part of the functionality as described above.

The computer 7 may be connected to an input device 92 such as mouse and/or keyboard and a display device 94 such as liquid crystal display, via corresponding I/O interfaces 80a and 80b as well as the system bus 82. In case the computer 7 is implemented as a tablet computer, for example, a touch panel that displays information and that receives input may be connected to the computer 7 via a corresponding I/O interface and the system bus 82. Further, in some examples, although not shown in Fig. 8, the computer 7 may further be connected to a printer and/or an imaging device such as a camera, via corresponding I/O interfaces and the system bus 82.

In addition or as an alternative to an implementation using a computer 7 as shown in Fig. 8, a part or all of the functionality of the exemplary embodiments described herein may be implemented as one or more hardware circuits. Examples of such hardware circuits may include but are not limited to: Large Scale Integration (LSI), Reduced Instruction Set Circuits (RISC), Application Specific Integrated Circuit (ASIC) and Field Programmable Gate Array (FPGA).

## Claims

1. A computer-implemented method comprising:
- determining a first sequence based on a plurality of sequence reads;
- displaying, on a display device, a user interface, UI, including at least one second sequence comprising one or more first sequence reads of the plurality of sequence reads, wherein when the first and second sequences are aligned, at least one of the first sequence reads comprises at least one position that differs from a corresponding position of the first sequence, and wherein the at least one position are displayed such that such a position is identifiable from the other positions where the first and the second sequences are identical; and
- upon receiving an indication that one of the at least one second sequences is selected via the UI, displaying on the UI information about matched one or more reference sequences that match the selected second sequence.

2. The computer-implemented method according to claim 1, wherein the first sequence is a consensus sequence constructed based on the plurality of sequence reads.

3. The computer-implemented method according to claim 1, wherein the first sequence is a reference sequence identified based on querying a sequence database with each sequence read and selecting the most probable reference sequence within the sequence database based on the queries.

4. The computer-implemented method according to any one of the preceding claims, wherein the information about the matched one or more reference sequences comprises one or more of: reference sequence gene names or identifiers, match accuracy rate, or match error rate.

5. The computer-implemented method according to any one of the preceding claims, wherein those second sequences that have a number of differing positions higher than a threshold number are noise sequences of interest, the method further comprises automatically querying a sequence database with each of the noise sequences of interest to identify one or more reference sequences that match each noise sequence of interest and store information about the matched one or more reference sequences, and/or
a second sequence is a noise sequence of interest, if a position of the first sequence is identified as a noise position and the second sequence differs from the first sequence at the noise position, wherein a noise position is a position at which a noise level is above a predetermined noise threshold and/or a noise position is a position at which the distance of the noise level from a confidence level of the first sequence is below a predetermined distance threshold, wherein the method optionally further comprises automatically calculating the noise level of each position of the first sequence and displaying at least a part of results of automatically calculating the noise level of each position of the first sequence such that one or more positions of the first sequence that are identified as noise positions are identifiable from other positions of the first sequence that are not identified as noise positions.

6. The computer-implemented method according to claim 5, wherein displaying comprises upon receiving indication that a noise sequence of interest is selected, identify and display the stored information associated with the selected noise sequence of interest.

7. The computer-implemented method according to any one of claims 1 to 4, wherein those second sequences that have a number of differing positions higher than a threshold number are noise sequences of interest, the method further comprises upon receiving the indication, automatically querying a sequence database with the selected noise sequence of interest to identify one or more reference sequences that match the noise sequence of interest.

8. The computer-implemented method according to any one of claims 1 to 4, further comprising:
upon receiving the indication that one of the at least one second sequence is **selected** via the UI, querying a sequence database with the selected second sequence to determine the matched one or more reference sequences that match the selected second sequence.

9. The computer-implemented method according to any one of the preceding claims, wherein the at least one position that differs from the corresponding position of the first sequence is a result of:
a measurement and/or analysis noise when creating the first sequence reads from one or more samples; and/or
the one or more samples.

10. The computer-implemented method according to any one of the preceding claims, wherein an instruction to display on the UI information about the matched one or more reference sequences that match the selected second sequence is further received in connection with the indication that one of the at least one second sequence is selected via the UI, and
wherein, optionally, the instruction is received via a trigger element that is:
a permanent element that is present within the UI during a whole time in which the UI is displayed on the display device, or
a temporary element that appears within the UI in response to a particular user input with respect to the selected sequence read.

11. The method according to any one of the preceding claims, wherein displaying on the UI the information about the matched one or more reference sequences that match the selected second sequence comprises ranking the matched one or more reference sequences based on a percentage of a match of each one of the matched one or more reference sequences with respect to the selected second sequence,
wherein, optionally, identifiers for each of the matched one or more reference sequences are displayed in an order according to the ranking,
wherein, optionally, the step of ranking the matched one or more reference sequences based on a percentage of a match of each one of the matched one or more reference sequences with respect to the selected second sequence comprises:
o determining the percentage of the match of each one of the matched one or more reference sequences with respect to the selected second sequence and, optionally, determining an error rate for each one of the matched one or more reference sequences with respect to the match with the selected second sequence, and
wherein, optionally, the percentage of the match and/or the error rate for each one of the one or more reference sequences is further provided.

12. The method according to any one of the preceding claims, wherein the reference sequences are human leukocyte antigen, HLA, alleles, and/or wherein the sequence database is an IPD-MHC database, and/or wherein the plurality of sequence reads is obtained from a single sample.

13. The method according to any one of the preceding claims, further comprising:
grouping the at least one second sequence in a specified order for display in the UI,
wherein the grouping is performed automatically or in response to a user input to trigger the grouping.

14. A computer program product comprising computer-readable instructions that, when loaded and run on a computer, cause the computer to perform the method according to any one of the preceding claims.

15. A device comprising:
one or more processors configured to perform the method according to any one of claims 1 to 13.
